(19) Europäisches Patentamt
European Patent Office
Office européen des brevets

(11) Veröffentlichungsnummer : 0 088 328
B1

(12) # EUROPÄISCHE PATENTSCHRIFT

(45) Veröffentlichungstag der Patentschrift : 30.07.86

(51) Int. Cl.[4] : B 01 J 27/18, C 07 C 57/055

(21) Anmeldenummer : 83101939.3

(22) Anmeldetag : 28.02.83

(54) **Oxidationskatalysator, inbesondere für die Herstellung von Methacrylsäure durch Gasphasenoxidation von Methacrolein.**

(30) Priorität : 10.03.82 DE 3208571

(43) Veröffentlichungstag der Anmeldung : 14.09.83 Patentblatt 83/37

(45) Bekanntmachung des Hinweises auf die Patenterteilung : 30.07.86 Patentblatt 86/31

(84) Benannte Vertragsstaaten : BE DE FR GB IT NL

(56) Entgegenhaltungen :
EP-A- 0 088 327
DE-A- 2 624 134
DE-A- 2 644 107
FR-A- 2 040 546
GB-A- 1 473 035
GB-A- 2 001 256

(73) Patentinhaber : BASF Aktiengesellschaft
Carl-Bosch-Strasse 38
D-6700 Ludwigshafen (DE)

(72) Erfinder : Krabetz, Richard, Dr.
Unterer Waldweg 8
D-6719 Kirchheim (DE)
Erfinder : Schwarzmann, Matthias, Dr.
Carl-Bosch-Strasse 54
D-6703 Limburgerhof (DE)

EP 0 088 328 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Es sind zahlreiche Oxidationskatalysatoren und deren Verwendung zur Herstellung von Methacrylsäure durch Gasphasenoxidation von Methacrolein vorgeschlagen worden. Diese befriedigen jedoch nicht oder nur zum Teil die Anforderungen des technischen Betriebes hinsichtlich hoher Selektivität bei hohen Methacroleinumsätzen und hohen Raumbelastungen über lange Betriebszeiten.

Aus der GB-A-2 040 717 sind z. B. Katalysatoren bekannt, die Mo, Cu, P, Sb und Cs und/oder Ca enthalten. Mit diesen Katalysatoren werden jedoch nur unbefriedigende Selektivitäten von 76 % für die Methacrylsäurebildung bei Methacroleinumsätzen von nur 75 % erreicht. In der GB-A-1 473 035 werden Katalysatoren vorgeschlagen, die zusätzlich zu Mo, Cu und P wenigstens ein Alkalimetall, ausgewählt aus der Gruppe Li, Na, K, Rb, Cs, und wenigstens ein Metall, ausgewählt aus der Gruppe Sb, V, W, Fe, Mn, Sn enthalten. Mit diesen Katalysatoren werden zwar im Dauerbetrieb Umsätze des Methacrolein bis 91,5 % und Selektivitäten von 82 % erzielt, die niedrigen Durchsätze (space velocity) von 1 000 $h^{-1}$ und relativ hohen Temperaturen von 325 °C und mehr befriedigen jedoch nicht. Auch Oxidationskatalysatoren der aus der GB-A-2 046 252 bekannten Art, die Mo, P, V sowie ggf. As und Cu oder andere kationische Elemente enthalten, zeigen zwar hohe katalytische Wirksamkeit, jedoch nur bei technisch uninteressanten Katalysatorteilchengrößen unter 2 mm und verhältnismäßig hohen Temperaturen von 330 °C. Oxidationskatalysatoren, die in Gegenwart hoher Chlorionenkonzentrationen von etwa bis 5 Äquivalenten je Äquivalent Molybdän hergestellt werden, wie beispielsweise die Mo, P, W enthaltenden Katalysatoren der US-A-4 212 767 und 4 272 408 oder die Mo, P, Sb und gegebenenfalls W enthaltenden Katalysatoren gemäß der US-A-39 65 163 zeigen zwar eine verhältnismäßig gute katalytische Wirksamkeit bei kurzen Betriebszeiten, doch ist es schwierig, langlebige und ausreichend selektive Katalysatoren dieser Art reproduzierbar herzustellen. Außerdem neigen die genannten Katalysatoren zu verstärkter Bildung von Essigsäure, wenn sie in technisch sinnvollen Teilchengrößen von mindestens 3 mm eingesetzt werden. Aus der GB-A-2 001 256 sind weiter oxidische Katalysatoren bekannt, die Mo, P, As, Cu und Cr enthalten und die in Anwesenheit oder Abwesenheit einer dibasischen Carbonsäure, Oxycarbonsäure, Mannit oder Pyrogallol als Reduktionsmittel hergestellt sind.

Die Eigenschaften dieser und der obengenannten Katalysatoren sind im allgemeinen jedoch dann unbefriedigend, wenn bei den genannten Verfahren zur Herstellung von Methacrylsäure als Rohstoff Methacrolein eingesetzt wird, das durch Kondensation von Propanal mit Formaldehyd hergestellt ist. Dieses enthält als herstellungsbedingte Verunreinigungen neben nicht umgesetzten Propanal organische Amine, Dimere des Methacroleins und Methylpentenal, und bereits geringe Mengen dieser Verunreinigungen führen im allgemeinen zu einer mehr oder weniger starken Leistungsminderung der Katalysatoren der genannten Art.

Es bestand daher ein technisches Interesse, Oxidationskatalysatoren, insbesondere für die Oxidation von Methacrolein zu Methacrylsäure in der Gasphase, zu finden, die bei Einsatz technischer Methacroleinqualitäten und technisch bei Festbettreaktionen üblicher Teilchengrößen des Katalysators hohe Ausbeuten und eine geringe Bildung an Nebenprodukten auch bei hohen Raumbelastungen und langen Betriebszeiten gewährleisten. Diese Aufgaben können mit dem Oxidationskatalysator gemäß dieser Erfindung gelöst werden.

Es wurde nun gefunden, daß Oxidationskatalysatoren, die als Basiskomponenten neben Sauerstoff und gegebenenfalls $NH_4^+$-Ionen Molybdän, Wolfram, Phosphor und Antimon im Verhältnis der Atome Mo : P : W : Sb gleich 12 : 0,1-3 : 0,1-4 : 0,1-3 enthalten und die in an sich üblicher Weise durch Vereinigung einer Molybdän-Phosphor-, Antimon- und Wolfram-Verbindung in wäßriger Lösung oder Suspension, Abtrennen des Wassers und Calcinieren des Rückstandes bei 180 bis 400 °C hergestellt sind, besonders geeignet für Gasphasenreaktionen sind, wenn die Vereinigung bei einer Chlorionenkonzentration unter 0,3 Mol je Mol Molybdän sowie in Gegenwart von Ionen von Monocarbonsäuren mit 1 bis 2 C-Atomen, Dicarbonsäuren deren Konzentration 0,02 bis 2 Mol je Mol Molybdän beträgt durchgeführt wurde und deren Zusammensetzung durch die allgemeine Formel

$$Mo_{12}P_aW_bSb_cAs_dCu_eX_fY_gO_x$$

wiedergegeben wird, in der X für ein oder mehrere Elemente aus der Gruppe Nb, Mn, Fe, Sn und Cr, Y für K, Rb, Cs

a für 0,1 bis 3,
b für 0,1 bis 4,
c für 0,1 bis 3,
d für 0 bis 1,
e für 0 bis 1,
f für 0 bis 1,
g für 0 bis < 0,1,
$d + e \neq 0$
$e + f + g$ für $\leq 2$

und x für die zur Absättigung der Valenzen formal erforderliche Anzahl der Sauerstoffatome stehen. Die Carbonsäure-Ionen leiten sich vorzugsweise von Ameisensäure, Essigsäure, Oxalsäure und solchen Carbonsäuren ab, die sowohl als Reduktionsmittel wirken als auch mit Antimonverbindungen schwer hydrolysierbare, wasserlösliche Salze bzw. Komplexe bilden, insbesondere der Weinsäure und Zitronensäure. Ganz besonders bevorzugt ist die Ameisensäure oder Ameisensäure in Kombination mit den obengenannten anderen Carbonsäuren. Obwohl Katalysatoren, die lediglich Arsen oder Kupfer enthalten, bereits gute katalytische Wirksamkeit aufweisen, werden solche Katalysatoren bevorzugt, die Arsen und Kupfer in Mengen von $> 0$ bis 1 Atom je 12 Atome Mo enthalten. Insbesondere bevorzugt sind Oxidationskatalysatoren der allgemeinen Formel

$$Mo_{12}P_aW_bSb_cAs_dCu_eX_fY_gO_x$$

in der

X für ein oder mehrere Elemente aus der Gruppe Nb, Mn, Fe, Sn und Cr,
Y für K, Rb, Cs,
a für 0,1 bis 3,
b für 0,1 bis 4,
c für 0,1 bis 3,
d für $> 0$ bis 1,
e für $> 0$ bis 1,
f für 0 bis 1,
g für 0 bis $< 0{,}1$,
$e + f + g$ für $\leq 2$ und
x für die Absättigung der Valenzen der anderen Katalysatorbestandteile formal erforderliche Anzahl der Sauerstoffatome. Die gegebenenfalls vorhandenen $NH_4^+$-Ionen sind in die Formel aus formalen Gründen nicht mit aufgenommen worden.

Die Anwesenheit von Alkalimetallen ist an sich unerwünscht, da Alkalimetalle bei höheren Temperaturen die Bildung der inaktiven $MoO_3$-Phase begünstigen. Vorzugsweise soll die Menge an K, Rb und Cs im Katalysator 0,035 Atome je 12 Atome Molybdän nicht überschreiten. Von den Komponenten X in der oben genannten Formel wird Nb und/oder Fe gegebenenfalls in Kombination mit einem der anderen Elemente der Gruppe X vorgezogen. Hinsichtlich der Zusammensetzung werden solche Oxidationskatalysatoren der oben genannten Formel bevorzugt, in denen

$a = 0{,}5\text{-}2$,
$b = 0{,}5\text{-}3$,
$c = 0{,}2\text{-}1{,}5$,
$d = 0{,}05\text{-}0{,}5$,
$e = 0{,}05\text{-}0{,}8$, insbesondere $0{,}1\text{-}0{,}5$,
$f = 0\text{-}0{,}8$, insbesondere $> 0\text{-}0{,}5$
$g = 0\text{-} < 0{,}035$ und
$e + f + g \leq 1$ sind.

Die Katalysatorkomponenten können gegebenenfalls als Oxide bzw. Säureanhydride eingesetzt werden, vorgezogen werden jedoch im allgemeinen wasserlösliche Verbindungen. Geeignete Molybdän- und Wolframverbindungen sind beispielsweise Ammoniummolybdat, Phosphomolybdänsäuren und ihre Salze, Ammoniumwolframat, Phosphowolframsäure und ihr Ammoniumsalz. Obwohl auch andere Salze eingesetzt werden können, werden die genannten Verbindungen bevorzugt, insbesondere Ammoniummolybdat oder Phosphomolybdänsäure als Molybdän- und Phosphowolframsäure als Wolframverbindung. Als Phosphorquelle können neben den genannten Heteropolysäuren mit Vorteil Phosphorsäure und ihre Ammoniumsalze eingesetzt werden. Antimon und die kationischen Elemente können in Form ihrer Chloride eingesetzt werden, doch soll die Gesamtmenge an Chlorionen bei der Herstellung der Katalysatoren unter 0,3 Mol, insbesondere unter 0,25 Mol je Mol Molybdän liegen und freie Chlorwasserstoffsäure nicht mitverwendet werden. Die kationischen Elemente Cu, Mn, Sn, Fe, Nb und Cr können in verschiedener Form, z. B. als Oxide, Hydroxide oder Carbonate, wie beispielsweise $Nb_2O_3$, Sn(II)O, $Cu(OH)_2xCuCO_3$, oder als Formiate, Oxalate oder Acetate eingesetzt werden. Derartige Salze sind gegenüber Nitraten bevorzugt. Arsen wird vorteilhaft als Ammoniumarsenat oder Arsensäure zugegeben, obwohl auch andere Verbindungen möglich sind.

Die erfindungsgemäßen Katalysatoren können im allgemeinen in der Weise hergestellt werden, daß die vereinigten Lösungen der Molybdän-, Phosphor- und gegebenenfalls Arsenverbindung zunächst mit der Antimonverbindung, die vorteilhaft in der wäßrigen Lösung der Carbonsäuren gelöst oder fein dispergiert ist, und anschließend mit der wäßrigen Lösung der Wolframverbindung vereinigt werden. Die gegebenenfalls zusätzlich eingesetzten Komponenten werden im allgemeinen nach Vereinigung der Hauptkomponente zugesetzt. Die Menge der Carbonsäure beträgt im allgemeinen 0,02 bis 2 Mol,

vorzugsweise 0,05 bis 1,5 Mol je Mol Molybdän. Die Carbonsäuren können mit Vorteil als solche zugegeben werden, wobei die Ameisensäure, Essigsäure, Weinsäure und Zitronensäure, insbesondere jedoch die Ameisensäure allein oder in Kombination mit den anderen Carbonsäuren bevorzugt sind. Durch die Carbonsäureionen wird die Bildung einer besonders stabilen Form der phosphorhaltigen Heteropolysäure und ihrer Salze gefördert, die nach Trocknung, Verformung und Calcinierung eine katalytisch besonders wirksame Struktur des aktiven Katalysators ergibt.

Die Trocknung der wäßrigen Lösung oder Suspension der Komponenten geschieht im allgemeinen durch Eindampfen in Rührkesseln bei Temperaturen unter 140 °C oder durch Sprühtrocknung bei Austrittstemperaturen zwischen 80 und 140 °C.

Nach dem Trocknen werden die erhaltenen Massen meist auf Teilchengrößen von 200 bis 1 200 µm gemahlen und gegebenenfalls nach Zusatz üblicher Träger, wie $SiO_2$ oder Aluminiumoxiden, sowie gegebenenfalls von Gleitmitteln, wie Graphit, zu Kugeln, Tabletten, Ringen oder anderen Formen verpreßt. Man kann dann anschließend in der Luft, unter Stickstoff oder schwach reduzierender Atmosphäre bei geringen Gasströmungen und Temperaturen von 180 bis 400 °C, vorzugsweise von 220 bis 380 °C, insbesondere von 320 bis 360 °C calcinieren bzw. aktivieren. Die Trägerstoffe können auch während des Eindampfens der Katalysatorsuspension zugegeben werden, wodurch die Katalysatorkomponenten auf den Trägern abgeschieden werden. Schließlich können auch die getrockneten und gemahlenen Katalysatormassen ohne Zusatz von Trägern bei den genannten Temperaturen calciniert und anschließend zu geformten Gebilden verarbeitet oder auf Träger, insbesondere auf kugelförmige Träger in Form von Schalen in an sich üblicher Weise, z. B. nach den aus den US-A-4 305 843 und 4 297 247 bekannten Verfahren aufgebracht werden. Die katalytisch aktiven Massen weisen nach der Calcinierung ausschließlich die Struktur einer gestörten Heteropolysäure oder ihrer Salze mit charakteristischen Röntgenbeugungslinien auf. Sie sind besonders für die Oxidation von Methacrolein zu Methacrylsäure unter an sich üblichen Bedingungen in der Gasphase geeignet, insbesondere dann, wenn als Ausgangsmaterial Methacrolein verwendet wird, das durch Kondensation von Formaldehyd mit Propanal hergestellt ist.

Bei der Gasphasenoxidation von Methacrolein, die Gegenstand der europäischen Patentanmeldung EP-A-88 327 ist, werden als Oxidationsmittel Sauerstoff und Wasserdampf enthaltende Gasgemische eingesetzt, die über im allgemeinen fest in Katalysatorbetten angeordnete Katalysatoren geleitet werden. Man arbeitet dabei im allgemeinen bei Drücken von 1 bis 5 bar, vorteilhaft von 1 bis 2,5 bar. Im allgemeinen beträgt bei dem Verfahren die auf Standardbedingungen bezogene Verweilzeit der Methacrolein enthaltenden Gasgemische 0,5 bis 5 sec. und Verweilzeiten von 1 bis 3 sec. bei Temperaturen im Bereich von 200 bis 340 °C, insbesondere bei 220 bis 320 °C, werden vorgezogen. Zusätzlich zu Sauerstoff, Methacrolein und Wasserdampf enthalten die Reaktionsgase im allgemeinen indifferente Gase, insbesondere Stickstoff, und der Sauerstoff wird im allgemeinen als Luft zugeführt. Er kann jedoch auch als Reinsauerstoff eingesetzt werden. Zudem enthält das Reaktionsgas im allgemeinen Kohlenoxide, insbesondere dann, wenn nach der Abtrennung der entstandenen Methacrylsäure restliches Reaktionsabgas als Verdünnungsmittel zusammen mit nichtumgesetztem Methacrolein in die Oxidationsreaktion zurückgeführt wird.

Im Reaktionsgas beträgt das molare Verhältnis von Methacrolein : Sauerstoff : Wasser : Inertgas meist 1 : 1 bis 6 : 1 bis 20 : 4 bis 50, vorzugsweise 1 : 1,5 bis 4 : 2, bis 10 : 6 bis 30. Die Abtrennung der Methacrylsäure von den heißen Reaktionsabgasen kann in an sich üblicher Weise, meist durch Abschrecken mit Wasser durchgeführt werden.

Das Methacrolein kann nach verschiedenen Verfahren gewonnen sein, z. B. durch Gasphasenoxidation von tert. Butyl-alkohol, Isobutylen oder $C_4$-Gemischen oder durch Kondensation von Propionaldehyd mit Formaldehyd. Der Einsatz der erfindungsgemäßen Katalysatoren ist von besonderem Vorteil, wenn Methacrolein eingesetzt wird, das durch Kondensation von Propionaldehyd mit Formaldehyd in Gegenwart von Salzen sekundärer Amine oder mit Aminalen in Gegenwart von Säuren in wäßriger Lösung hergestellt ist. Technische Qualitäten, die auf diese Weise hergestellt sind, haben im allgemeinen einen Reinheitsgrad von 94 bis 99 % und enthalten neben nicht umgesetztem Propionaldehyd geringe Mengen organische Amine, wie Diethylamin oder Diethanolamin, Methylpentenal und Dimere des Methacroleins. Die Reinheitsangaben beziehen sich auf wasserfreies Rohmethacrolein, das bis zu 3,5 Gew.% Wasser enthalten kann. Soweit nichtumgesetztes Methacrolein und nicht kondensierte Reaktionsabgase in die Oxidationsreaktion zurückgeführt werden, kann das Synthesegasgemisch auch geringe Mengen leicht flüchtiger Nebenprodukte, wie Kohlenoxide oder Acrolein enthalten.

Bei der technischen Durchführung des Verfahrens arbeitet man meist in sogenannten Rohrbündelreaktoren, in denen die Katalysatoren fest angeordnet sind. Zur Vermeidung von örtlichen Überhitzungen kann die Katalysatoraktivität derart verändert werden, daß sie im Reabtionsrohr in Strömungsrichtung der Reaktionsgase stetig oder in Stufen ansteigt. Dies kann z. B. durch Verdünnen des Katalysators mit weniger aktiven oder inaktiven Katalysator- oder Trägerformlingen oder durch Einsatz von 2 oder mehr Katalysatoren mit unterschiedlicher Aktivität und/oder Selektivität erfolgen. Es ist auch möglich, die erfindungsgemäße Oxidation des Methacroleins zu Methacrylsäure in der Wirbelschicht durchzuführen, fest angeordnete Katalysatorschichten werden jedoch vorgezogen.

Bei der Aufarbeitung der Reaktionsgase, bei der vor dem Waschen mit Wasser auch indirekt gekühlt werden kann, werden wäßrige Lösungen der Methacrylsäure erhalten, die gegebenenfalls zusätzlich

geringe Mengen Essigsäure, Maleinsäure und Acrylsäure enthalten. Aus den erhaltenen Lösungen der Methacrylsäure kann diese mit geeigneten Lösungsmitteln, wie beispielsweise Methylmethacrylat in an sich üblicher Weise extrahiert und entweder direkt mit Alkanolen verestert oder durch Destillation aus dem Extrakt abdestilliert und von den Nebenprodukten getrennt werden. Das nichtumgesetzte Methacrolein kann aus dem wäßrigen Kondensat destilliert oder z. B. mit Wasserdampf ausgetrieben und der Oxidationsreaktion wieder zugeführt werden.

Die erfindungsgemäßen Katalysatoren zeigen auch bei anderen Oxidationsreaktionen, z. B. bei der Oxidation von Acrolein zu Acrylsäure oder bei der Oxidation von substituierten Toluolderivaten zu substituierten Benzaldehyden und Benzoesäuren gute Wirksamkeit und Selektivität.

In den folgenden Beispielen wird zur Prüfung der Katalysatoren Methacrolein eines Reinheitsgrades von 97 bis 99 % eingesetzt, das neben Wasser und Propionaldehyd geringe Mengen an sekundären Aminen und Nebenprodukten der Methacroleinsynthese aus Propionaldehyd und Formaldehyd enthält. Die darin angegebenen Teile und Prozente beziehen sich, soweit nicht anders angegeben, auf das Gewicht. Die darin angegebenen Volumenteile verhalten sich zu den Gewichtsteilen wie das Liter zum Kilogramm.

Beispiel 1

Eine wäßrige Lösung von 212 Teilen Ammoniumheptamolybdat in 600 Teilen Wasser wird nacheinander mit einer Lösung von 13,2 Teilen Di-Ammoniumphosphat und 1,3 Teilen Di-Arsenpentoxidhydrat in 100 Volumen-Teilen Wasser, von 22,6 Teilen Antimontrichlorid in einer Mischung von 6 Volumen-Teilen Ameisensäure und 20 Volumen-Teilen Wasser, einer Lösung von 21 Teilen Wolframatophosphorsäure in 50 Volumen-Teilen Wasser und schließlich einer Lösung von 2,5 Teilen Kupfer-(II)-acetat in 100 Volumen-Teilen Wasser versetzt. Nach dem Eindampfen der erhaltenen Suspension wird die getrocknete Masse auf einen Teilchendurchmesser von 0,4 bis 1,2 mm gemahlen und nach Zusatz von 2 % Graphitpulver zu 3 × 3 mm Tabletten verpreßt. Zur Aktivierung werden die Tabletten 6 Stunden an der Luft auf 355 °C erhitzt. Der Katalysator hat die formale Zusammensetzung

$$Mo_{12}W_{0,9}P_{1,09}As_{0,1}Sb_1Cu_{0,12}O_X$$

80 Volumenteile Katalysatortabletten werden in ein Salzbad-beheiztes Reaktionsrohr von 16 mm Durchmesser gefüllt. Über den Katalysator wird mit einer Raumgeschwindigkeit von 1 320 Nl/l/Stunde eine Gasmischung aus 3,3 Volumenprozent Methacrolein, 9,1 Volumenprozent Sauerstoff, 29,5 Volumenprozent Wasserdampf und 58,1 Volumenprozent Stickstoff geleitet. Bei einer Badetemperatur von 318 °C beträgt der Methacroleinumsatz nach 7 Tagen 94,3 Mol.-%, die Selektivität 85,8 Mol.-% und die Methacrylsäureausbeute 80,9 Mol.-%. Nach 30 Tagen beträgt der Umsatz 94,1 Mol.-%, die Selektivität 86,2 Mol.-% und die Ausbeute 81,1 Mol.-%. Die Ausbeute an Essigsäure betrug 2,6 %.

Beispiele 2 bis 13

Entsprechend Beispiel 1 werden verschiedene Oxidations-katalysatoren mit unterschiedlichen Zusammensetzungen hinsichtlich der Zusatzkomponenten zu den Basiskomponenten Mo, P, W und Sb hergestellt. Die Zusammensetzungen sind in der folgenden Tabelle 1 zusammen mit den Testergebnissen aufgeführt. Die Zusatzkomponenten wurden in Form folgender Salze eingesetzt :

Mangan(II)acetat, Eisen(II)oxalat, Ammoniumchromat, Niob(V)oxid, Zinn(II)oxid

(Siehe Tabelle 1 Seite 6 f.)

Tabelle 1

| Beispiel | Katalysator | Bad-temp. °C | Umsatz Mol.-% | Selektivität Mol.-% | Ausbeute Mol.-% | nach Betriebstagen |
|---|---|---|---|---|---|---|
| 2 | $Mo_{12}P_{1,18}W_{1,8}Sb_{1,2}As_{0,1}Cu_{0,25}$ | 319 | 93,8 | 83 | 77,9 | 12 |
| 3 | $Mo_{12}P_{1,1}W_{0,9}Sb_{0,2}As_{0,2}Cu_{0,05}$ | 314 | 92,1 | 85 | 78,3 | 8 |
| 4 | $Mo_{12}P_{1,1}W_{0,9}Sb_{1}Cu_{0,25}$ | 284 | 91,7 | 84,6 | 77,5 | 6 |
| 5 | $Mo_{12}P_{1,1}W_{0,9}Sb_{1}As_{0,2}$ | 302 | 89,6 | 81,3 | 72,8 | 10 |
| 6 | $Mo_{12}P_{1,1}W_{0,9}Sb_{1}As_{0,2}Cu_{0,1}Mn_{0,1}$ | 318 | 91,7 | 84,5 | 77,4 | 10 |
| 7 | $Mo_{12}P_{1,1}W_{0,9}Sb_{1}As_{0,2}Cu_{0,1}Nb_{0,4}$ | 324 | 95 | 87,2 | 82,8 | 7 |
| 8 | $Mo_{12}P_{1,1}W_{0,9}Sb_{1}As_{0,2}Cu_{0,1}Fe_{0,1}$ | 312 | 94,4 | 85,9 | 81,1 | 11 |
| 9 | $Mo_{12}P_{1,1}W_{0,9}Sb_{1}As_{0,2}Cu_{0,1}Sn_{0,05}$ | 316 | 91,1 | 84,0 | 76,5 | 8 |
| 10 | $Mo_{12}P_{1,1}W_{0,9}Sb_{1}As_{0,2}Cu_{0,1}$ | 316 | 92,9 | 85,2 | 79,2 | 10 |
| 11 | $Mo_{12}P_{1,2}W_{1,8}Sb_{1}As_{0,2}Cu_{0,25}K_{0,03}$ | 319 | 95,6 | 81,9 | 78,3 | 7 |
| 12 | $Mo_{12}P_{1,1}W_{0,9}Sb_{1}As_{0,2}Cu_{0,25}Cs_{0,09}$ | 326 | 92,2 | 81,9 | 75,6 | 4 |
| 13 | $Mo_{12}P_{11}W_{0,9}Sb_{1}As_{0,2}Cu_{0,25}Rb_{0,03}$ | 319 | 91,5 | 83,5 | 76,4 | 7 |

Beispiel 14

Wie in Beispiel 1 wurde ein Katalysator hergestellt mit der Änderung, daß anstelle des Antimontrichlorids Antimon(III)-oxid in einer Menge von 14,6 Teilen eingesetzt wurde. Unter den Testbedingungen des Beispiels 1, jedoch bei einer Badtemperatur von 284 °C, betrug der Umsatz 84,8 Mol.-%, die Selektivität 83,7 Mol.-%, die Methacrylsäureausbeute 71 Mol.-% und die Ausbeute an Essigsäure 0,86 Mol.-%.

Beispiel 15 bis 17

Entsprechend Beispiel 1 wurden weitere Katalysatoren hergestellt und getestet, mit der Änderung, daß statt der Ameisensäure Weinsäure, Zitronensäure und Oxalsäure eingesetzt wurden. Die zugesetzten Mengen und die Test-ergebnisse sind in der Tabelle 2 zusammengefaßt.

Tabelle 2

| Beispiel | Zusatz | Mole je Mol Mo | Badtemp. °C | Umsatz Mol.-% | Selektivität Mol.-% | Ausbeute Mol.-% |
|---|---|---|---|---|---|---|
| 15 | Weinsäure | 0,4 | 312 | 87,4 | 84,5 | 73,8 |
| 16 | Zitronensäure | 0,08 | 300 | 83,5 | 85,2 | 71,2 |
| 17 | Oxalsäure | 0,18 | 316 | 81 | 93,2 | 75,5 |

**Patentansprüche**

1. Oxidationskatalysator, der neben Sauerstoff- und ggf. $NH_4^+$-Ionen als Basiskomponenten Molybdän, Wolfram, Phosphor und Antimon im Verhältnis der Atome Mo : P : W : Sb gleich 12 : 0,1-3 : 0,1-4 : 0,1-3 enthält und der in an sich üblicher Weise durch Vereinigung einer Molybdän-, Phosphor-, Antimon- und Wolfram-Verbindung in wäßriger Lösung oder Suspension, Abtrennen des Wassers und Calcinieren des Rückstandes bei 180 bis 400 °C hergestellt ist, dadurch gekennzeichnet, daß die Vereinigung bei einer Chlorionenkonzentration unter 0,3 Mol je Mol Molybdän und in Gegenwart von Ionen von Monocarbonsäuren mit 1 bis 2 C-Atomen, Dicarbonsäuren oder Oxycarbonsäuren, deren Konzentration 0,02 bis 2,0 Mol je Mol Molybdän beträgt, durchgeführt wurde und daß die Zusammensetzung des Katalysators durch die allgemeine Formel

$$Mo_{12}P_aW_bSb_cAs_dCu_eX_fY_gO_x$$

wiedergegeben wird, in der X für ein oder mehrere Elemente aus der Gruppe Nb, Mn, Fe, Sn und Cr, Y für K, Rb, Cs
a für 0,1 bis 3,
b für 0,1 bis 4,
c für 0,1 bis 3,
d für 0 bis 1,
e für 0 bis 1,
f für 0 bis 1,
g für 0 bis $< 0,1$,
$e + f + g$ für $\leq 2$,
$d + e \neq 0$
und x für die zur Absättigung der Valenzen formal erforderliche Anzahl der Sauerstoffatome stehen.

2. Oxidationskatalysator nach Anspruch 1, dadurch gekennzeichnet, daß die Carbonsäureionen sich von der Ameisen-, Essig-, Oxal-, Wein- und/oder Zitronensäure ableiten.

3. Oxidationskatalysator nach Anspruch 1 oder 2, dadurch gekennzeichnet, daß eine Zusammensetzung durch die allgemeine Formel

$$Mo_{12}P_aW_bSb_cAs_dCu_eX_fY_gO_x$$

wiedergegeben wird, in der X für ein oder mehrere Elemente aus der Gruppe Nb, Mn, Fe, Sn und Cr

Y für K, Rb, Cs,
a für 0,1 bis 3,
b für 0,1 bis 4,
c für 0,1 bis 3,
d für $>0$ bis 1,
e für $>0$ bis 1,
f für 0 bis 1,
g für 0 bis $<0{,}1$,
$e+f+g$ für $\leq 2$ und
x für die zur Agsättigung der Valenzen formal erforderliche Anzahl der Sauerstoffatome stehen.

4. Verfahren zur Herstellung von Oxidationskatalysatoren gemäß Anspruch 2 durch Vereinigen von wäßrigen Lösungen oder Suspensionen von Molybdän-, Phosphor-, Antimon- und Wolframverbindungen sowie gegebenenfalls von üblichen Trägerstoffen, Abtrennen des Wassers aus den erhaltenen Gemischen und Calcinieren der Rückstände bei Temperaturen von 180 bis 400 °C, dadurch gekennzeichnet, daß bei der Vereinigung der Komponenten die Chlorionen-Konzentration des wäßrigen Mediums unter 0,3 Mol je Mol Molybdän liegt und je Mol Molybdän 0,02 bis 2 Mol Ameisen-, Essig-, Oxal-, Wein- und/oder Zitronensäure zugegeben werden.

**Claims**

1. An oxidation catalyst which, in addition to oxygen ions and optiomal $NH_4^+$ ions, contains molybdenum, tungsten, phosphorus and antimony as basic components in the atomic ratio Mo : P : W : Sb = 12 : 0.1-3 : 0.1-4 : 0.1-3 and which is prepared in a conventional manner by combining molybdenum, phosphorus, antimony and tungsten compounds in aqueous solution or suspension, removing the water and calcining the residue, wherein combination is carried out at a chloride ion concentration of less than 0.3 mole per mole of molybdenum and in the presence of ions of monocarboxylic acids of 1 or 2 carbon atoms, dicarboxylic acids or hydroxycarboxylic acids, the concentration of which is from 0.02 to 2 moles per mole of molybdenum, and wherein the composition of the catalyst is represented by the general formula

$$Mo_{12}P_aW_bSb_cAs_dCu_eX_fY_gO_x$$

where X is one or more elements from the group Nb, Mn, Fe, Sn and Cr, Y is K, Rb or Cs,
a is 0.1 to 3,
b is 0.1 to 4,
c is 0.1 to 3,
d is 0 to 1,
e is 0 to 1,
f is 0 to 1,
g is 0 to $<0.1$,
$e+f+g$ is $\leq 2$,
$d+e$ is $>0$, and
x is the number of oxygen atoms formally required to saturate the valencies of the other catalyst constituents.

2. An oxidation catalyst as claimed in claim 1, wherein the carboxylic acid ions are derived from formic, acetic, oxalic, tartaric and/or citric acid.

3. An oxidation catalyst as claimed in claim 1 or 2, the composition of which is represented by the general formula

$$Mo_{12}P_aW_bSb_cAs_dCu_eX_fY_gO_x$$

where X is one or more elements from the group Nb, Mn, Fe, Sn and Cr,

Y is K, Rb or Cs,
a is 0.1 to 3,
b is 0.1 to 4,
c is 0.1 to 3,
d is $>0$ to 1,
e is $>0$ to 1,
f is 0 to 1,
g is 0 to $<0.1$,
$e+f+g$ is $\leq 2$, and

x is the number of oxygen atoms formally required to saturate the valencies of the other catalyst constituents.

4. A process for the preparation of an oxidation catalyst as claimed in claim 2 by combining aqueous solution or suspensions of molybdenum, phosphorus, antimony and tungsten compounds, with or without conventional carriers, removing the water from the resulting mixture and calcining the residue at from 180 to 400 °C, wherein, on combining the components, the chloride ion concentration of the aqueous medium is less than 0.3 mole per mole of molybdenum, and from 0.02 to 2 moles of formic, acetic, oxalic, tartaric and/or citric acid are added per mole of molybdenum.

**Revendications**

1. Catalyseur d'oxydation contenant, à côté d'ions oxygène et éventuellement d'ions $NH_4^+$, du molybdène, du tungstène, du phosphore et de l'antimoine comme composants de base avec un rapport entre les atomes Mo : P : W : Sb égal à 12 : 0,1-3 : 0,1-4 : 0,1-3, préparé de manière usuelle par la réunion de composés du molybdène, du phosphore, de l'antimoine et du tungstène en solution ou suspension aqueuse, élimination de l'eau et calcination du résidu entre 180 et 400 °C, caractérisé en ce que la réunion est effectuée en présence d'ions chlore en une concentration inférieure à 0,3 mole par mole de molybdène et en présence d'ions d'acides mono-carboxyliques en $C_1$ ou $C_2$, d'acides di-carboxyliques ou d'acides oxycarboxyliques en une concentration de 0,02 à 2,0 moles par mole de molybdène et la composition du catalyseur correspond à la formule générale

$$Mo_{12}P_aW_bSb_cAs_dCu_eX_fY_gO_x$$

dans laquelle X représente un ou plusieurs éléments du groupe Nb, Mn, Fe, Sn et Cr et Y = K, Rb, Cs,
a vaut 0,1 à 3,
b vaut 0,1 à 4,
c vaut 0,1 à 3,
d vaut 0 à 1 et
e vaut 0 à 1 avec $d + e \neq 0$,
f vaut 0 à 1 et
g vaut 0 à $< 0,1$ avec $e + f + g \leq 2$, et
x désigne le nombre d'atomes d'oxygène nécessaires pour la saturation des valences dans cette formule.

2. Catalyseur d'oxydation suivant la revendication 1, caractérisé en ce que les ions d'acides carboxyliques dérivent de l'acide formique, de l'acide acétique, de l'acide oxalique, de l'acide tartrique et(ou) de l'acide citrique.

3. Catalyseur d'oxydation suivant la revendication 1 ou 2, caractérisé en ce que sa composition correspond à la formule générale

$$Mo_{12}P_aW_bSb_cAs_dCu_eX_fY_gO_x$$

dans laquelle X représente un ou plusieurs éléments du groupe Nb, Mn, Fe, Sn et Cr, Y = K, Rb, Cs,

a vaut 0,1 à 3,
b vaut 0,1 à 4,
c vaut 0,1 à 3,
d vaut $> 0$ à 1,
e vaut $> 0$ à 1,
f vaut 0 à 1 et
g vaut 0 à $< 0,1$ avec $e + f + g \leq 2$, et
x désigne le nombre d'atomes d'oxygène nécessaires pour la saturation des valences dans cette formule.

4. Procédé de préparation de catalyseurs d'oxydation suivant la revendication 2 par réunion de solutions ou suspensions aqueuses de dérivés du molybdène, du phosphore, de l'antimoine et du tungstène, ainsi que de matières supports usuelles éventuelles, élimination de l'eau des mélanges obtenus et calcination des résidus à des températures comprises entre 180 et 400 °C, caractérisé en ce que la réunion des composants est effectuée en présence d'une concentration du milieu aqueux en ions chlore inférieure à 0,3 mole par mole de molybdène et en ajoutant par mole de molybdène entre 0,02 et 2 moles d'acide formique, d'acide acétique, d'acide oxalique, d'acide tartrique et(ou) d'acide citrique.